# EUROPÄISCHE PATENTSCHRIFT

(11) **EP 0 873 302 B1**
(45) Veröffentlichungstag und Bekanntmachung des Hinweises auf die Patenterteilung: **27.09.2000**
(21) Anmeldenummer: 96938036.9
(22) Anmeldetag: 30.10.1996
(51) Int. Cl.: C07C 231/02, C07C 233/47, C07C 233/49, B01J 19/24, B01J 19/00

(54) **VERFAHREN ZUR KONTINUIERLICHEN HERSTELLUNG VON N-ACYLAMINOCARBONSÄUREN UND N-ACYLAMINOSULFONSÄUREN SOWIE DEREN ALKALIMETALLSALZEN**
PROCESS FOR THE CONTINUOUS PREPARATION OF N-ACYLAMINOCARBOXYLIC ACIDS AND N-ACYLAMINOSULPHONIC ACIDS AND THEIR ALKALI METAL SALTS
PROCEDE DE PREPARATION CONTINUE D'ACIDES N-ACYLAMINOCARBOXYLIQUES ET D'ACIDES N-ACYLAMINOSULFONIQUES, AINSI QUE LEURS SELS DE METAUX ALCALINS

(30) Priorität: 31.10.1995 DE 19540645
(43) Veröffentlichungstag der Anmeldung: 28.10.1998
(73) Patentinhaber: BASF AKTIENGESELLSCHAFT, 67056 Ludwigshafen (DE)
(72) Erfinder: EHLE, Beate, D-67071 Ludwigshafen (DE); SCHUH, Georg, D-67067 Ludwigshafen (DE); AUS DEM KAHMEN, Martin, D-67071 Ludwigshafen (DE); HERTEL, Dieter, D-69181 Leimen (DE)
(74) Vertreter: Isenbruck, Günter, Dr.
(86) Internationale Anmeldenummer: EP9604723
(87) Internationale Veröffentlichungsnummer: WO9716409

(56) Entgegenhaltungen:
- EP-A- 0 510 596
- DE-A- 1 493 660
- FR-A- 2 424 287
- GB-A- 692 568
- US-A- 4 095 952
- US-A- 4 278 539
- CHEMICAL ABSTRACTS, vol. 93, no. 16, 20.Oktober 1980 Columbus, Ohio, US; abstract no. 152017, KAJL, MARIAN ET AL: "A continuous method for making the acylation products of aminocarboxylic and aminosulfonic acids" XP002025188 in der Anmeldung erwähnt & TENSIDE DETERG. (1980), 17(4), 174-6, 1980,
- CHEMICAL ABSTRACTS, vol. 122, no. 5, 30.Januar 1995 Columbus, Ohio, US; abstract no. 56578, Seite 1250; XP002025189 & JP 06 256 276 A (KAO CORP.)

## Beschreibung

Die Erfindung betrifft ein Verfahren zur kontinuierlichen Herstellung von N-Acylaminocarbonsäuren und N-Acylaminosulfonsäuren sowie deren Alkalimetallsalzen aus den Alkalimetallsalzen von Aminocarbonsäuren bzw. Aminosulfonsäuren und Carbonsäurehalogeniden in einem als Umwälzkreis ausgebildeten Reaktor.

Zur Herstellung langkettiger Acylaminosäuren aus Fettsäurederivaten und Aminosäuren sind verschiedene Synthesewege bekannt, die sich im wesentlichen in der verwendeten Acylierungskomponente unterscheiden. Fettsäuren (DE-A 20 04 099) und Fettsäuremethylester (EP-A 0 033 392) sind zwar prinzipiell als Acylierungskomponente geeignet, konnten aber bislang keine großtechnische Bedeutung erlangen.

Ein industriell genutztes Verfahren zur Herstellung von langkettigen N-Acylaminosäuren ist die Kondensation von Fettsäurechloriden mit Aminosäuren unter Elimination von Salzsäure, auch bekannt als Schotten-Baumann-Reaktion. Dieses Verfahren ist seit langem bekannt (DE-C 635 522) und wird kommerziell genutzt, beispielsweise zur Herstellung von Oleoylsarkosin, auch bekannt unter der Bezeichnung "Medialansäure", die vor allem als Korrosionsinhibitor, Kraftstoffadditiv und in Lederhydrophobiermitteln Verwendung findet.

Das allgemeine Syntheseprinzip für Medialansäure, andere Acylsarkosinate und strukturverwandte Verbindungen (E. Jungermann et al., J. Amer. Chem. Soc. 78 (1956) 172 f; US 3,544,606, FR-B 1 465 959, DD-C 85 757, DE-A 14 93 650, DE-A 16 18 097) verläuft in der Weise, daß zunächst die Aminosäure als Na-Salz in wäßriger Lösung vorgelegt wird und daß dann unter Rühren Fettsäurechlorid und NaOH langsam so zudosiert werden, daß die Temperatur unter 35°C und der pH-Wert auf 9 - 12,5 gehalten wird. Nach beendeter Zugabe wird durch Zugabe von H₂SO₄ der pH-Wert des Reaktionsgemisches auf < 5 gestellt. Die so erhaltene Acylaminosäure wird als organische Phase abgetrennt, gegebenenfalls unter Zusatz eines organischen Lösungsmittels als Phasentrennmittel, das anschließend im Vakuum wieder entfernt wird. Falls das Alkalisalz der Acylaminosäure gewünscht wird, wird die Acylaminosäure in Wasser aufgenommen und durch Zugabe von NaOH neutralisiert. Zur Optimierung der einzelnen Verfahrensschritte bzw. Reaktionsparameter, z.B. der Phasentrennung oder der Verseifungsrate in Abhängigkeit von pH-Wert und Temperatur, wurden zahlreiche Schriften veröffentlicht.

Die auf den ersten Blick recht einfache Reaktion von Fettsäurehalogeniden mit Aminosäure-Salzen birgt einige kritische Punkte, deren sorgfältige Beachtung notwendig ist, um Acylaminosäuren in guter Ausbeute und hoher Reinheit herstellen zu können. Da die Kinetik der Reaktion sehr schnell ist, ist die Reaktion bei effektiver Durchmischung der beiden Reaktanden in < 1 min beendet. Im Alkalischen (pH > 8) ist somit die Acylierungsreaktion deutlich schneller (ca. 100 mal) als die in Konkurrenz ablaufende Verseifung des Fettsäurechlorides. Im sauren Milieu verlaufen beide Reaktionen etwa gleich schnell. Durch Temperaturerhöhung wird die Verseifung stärker beschleunigt als die Kondensation. Ferner ist die Acylierungsreaktion stark exotherm, das bedeutet: die schnelle Reaktionskinetik kann nur dann genutzt werden, wenn es gelingt, die bei der Reaktion freiwerdende Wärme rasch genug abzuführen. Da man im verdünnten wäßrigen Medium arbeitet, kann das Reaktionsgemisch zum Schäumen neigen (Tensid).

Die konventionelle, diskontinuierlich geführte Rührkesseltechnologie ist prinzipiell geeignet für die Herstellung von Acylaminosäuren, weist jedoch einige Nachteile auf, die letztendlich zu Lasten der Produktqualität gehen. Diese Nachteile sind: schwerfällige Temperaturführung und Wärmeabfuhr, ferner entsprechend der Chargenmenge relativ lange Verweilzeiten pro Ansatz. Mit fortschreitender Reaktion ändern sich die Eigenschaften des Reaktionsgemisches, z.B. Konzentration, Volumen, Viskosität, und damit auch die die Reaktionsgeschwindigkeit bestimmenden Größen.

Es ist daher auch schon vorgeschlagen worden, Abhilfe durch eine kontinuierliche Synthese zu schaffen. So ist bekannt (DE-A 14 93 660), zur kontinuierlichen Umsetzung von Fettsäurechloriden mit Aminocarbonsäuren bzw. Aminosulfonsäuren oder ihren löslichen Salzen zum Mischen und Reagierenlassen Mischgeräte zu verwenden, die aus feststehenden und rotierenden Teilen mit gezahnten, gelochten oder mit Stiften und ähnlichen Elementen versehenen Zylindern, Konen, Scheiben, Ringen oder Kränzen am rotierenden Teil und bzw. oder feststehenden Teil bestehen. Der Nachteil besteht darin, daß es sich bei solchen Rotor/Stator-Systemen um recht aufwendige und teure Apparate handelt. Eine effektive Wärmeabfuhr bis in den Bereich tieferer Temperatur (30 - 40°C, wie z.B. für Oleoylsarkosin günstig) ist schwierig, zumal durch die hohen Scherkräfte ein zusätzlicher Energieeintrag erfolgt.

M. Kajl et al, Tenside Detergents 17 (1980) 174 f, schlagen vor, zur effektiven Durchmischung der Reaktanden einen Düsenreaktor zu verwenden. Dies hat den Nachteil, daß die Wärmeabfuhr im Düsenreaktor nur schwer regelbar ist. Daher muß eine Kühlstrecke nachgeschaltet werden, und es kann zu lokalen Temperaturspitzen kommen. Des weiteren muß mit Schaumbildung gerechnet werden. Weiterhin ist bekannt (DD-C 131467), zur intensiven Durchmischung die Reaktandenströme dicht nebeneinander und unmittelbar unterhalb eines Rührsystems in einen Rührbehälter einzuführen. Hier treten dieselben Nachteile auf wie bei der diskontinuierlichen Rührkesseltechnologie.

Der Erfindung liegt deshalb die Aufgabe zugrunde, ein Verfahren zur kontinuierlichen Umsetzung von Fettsäurehalogeniden mit Aminocarbonsäuren und Aminosulfonsäuren bzw. deren Salzen zu finden, das kontinuierlich, in großen Mengen, in einer einfachen, kostengünstigen und vielseitig verwendbaren Apparatur, mit effektiver Wärmeabführung, ohne Schaumprobleme unter konstanten Bedingungen Acylaminosäuren in hoher Ausbeute und Reinheit liefert.

Diese Aufgabe wird erfindungsgemäß gelöst durch ein Verfahren zur kontinuierlichen Herstellung von N-Acylaminocarbonsäuren und N-Acylaminosulfonsäuren sowie deren Alkalimetallsalzen aus den Alkalimetallsalzen von Aminocarbonsäuren bzw. Aminosulfonsäuren und Carbonsäurehalogeniden in einem als Umwälzkreis ausgeführten Reaktor, bei dem die Reaktanden zur sofortigen Reaktion in den Umwälzkreis eingespeist und ein der Zulaufmenge entsprechender Teil der Produktlösung kontinuierlich aus dem Umwälzkreis ausgeschleust wird, die Reaktanden durch eine Umwälzpumpe im Kreislauf geführt werden, die bei der Reaktion gebildete Wärme abgeführt und die Temperatur in dem Reaktor geregelt wird.

Dem Kreislauf wird vorteilhafterweise zur Einhaltung eines konstanten pH-Werts pH-gesteuert alkalische Lösung zudosiert. Außerdem wird in den Reaktionskreislauf bei Bedarf ein organisches Phasentrennmittel und/oder ein schaumdämpfendes Mittel eingespeist.

Es wurde gefunden, daß die turbulente Strömung in einem einfachen Rohr ausreicht, um eine effektive Durchmischung von Fettsäurehalogenid und Aminosäuresalz-Lösung zu erzielen und so die schnelle Reaktionskinetik zu nutzen.

Das Verfahren kann in einer Vorrichtung durchgeführt werden, in der ein Reaktor, bestehend aus hintereinander angeordneten Rohren mit integrierter Umwälzpumpe, vorgesehen ist, dessen Auslaßende über ein Rohr mit seinem Einlaßende verbunden ist, und in der der Reaktor mit Zuführungsleitungen für die Reaktanden sowie mit einem Überstromventil für das Reaktionsprodukt versehen ist.

Für die Herstellung von N-Acylaminocarbonsäuren und N-Acylaminosulfonsäuren sowie deren Alkalimetallsalzen aus den Alkalimetallsalzen von Aminocarbonsäuren bzw. Aminosulfonsäuren und Carbonsäurehalogeniden läßt sich das erfindungsgemäße Verfahren besonders gut anwenden, wenn man als Alkalimetallsalze von Aminocarbonsäuren die Lithium-, Natrium- oder Kaliumsalze von aliphatischen Aminocarbonsäuren mit 2 bis 10 C-Atomen, vorzugsweise 3 bis 6 C-Atomen, insbesondere von Valin, Leucin, Norleucin, Glycin, Alanin, β-Alanin, ε-Aminocapronsäure, α-Aminoisobuttersäure, Sarkosin (N-Methylglycin), Asparaginsäure, Glutaminsäure oder Iminodiessigsäure einsetzt. Es lassen sich aber auch die Natrium- oder Kaliumsalze von anderen natürlichen α-Aminosäuren, von Oligopeptiden oder von aromatischen oder cycloaliphatischen Aminocarbonsäuren, z.B. Anthranilsäure, Phenylglycin, Phenylalanin oder 1-Aminocyclohexan-1-carbonsäure, verwenden. Dabei ist es unerheblich, ob die Alkalimetallsalze der Aminocarbon- bzw. -sulfonsäuren als solche eingesetzt oder im alkalischen Reaktionsmedium aus den entsprechenden Aminosäuren erzeugt werden.

Unter Aminocarbonsäuren sind hier vor allem Verbindungen mit einer primären oder sekundären Aminogruppe und einer oder zwei Carboxylgruppen pro Molekül zu verstehen; es können prinzipiell jedoch auch Verbindungen mit mehr als einer Aminogruppe und/oder mehr als zwei Carboxylgruppen eingesetzt werden, die Menge an Carbonsäurehalogeniden richtet sich dann nach der Anzahl der Aminogruppen. Alle Carboxylgruppen liegen praktisch vollständig in der Salzform vor.

Das erfindungsgemäße Verfahren läßt sich ebenfalls besonders gut anwenden, wenn man als Alkalimetallsalze von Aminosulfonsäuren die Lithium-, Natrium- oder Kaliumsalze von aliphatischen Aminosulfonsäuren mit 2 bis 10 C-Atomen, vorzugsweise 2 bis 4 C-Atomen, einsetzt. Insbesondere sind hier die entsprechenden Salze von Taurin (2-Aminoethansulfonsäure) und N-Methyltaurin von Interesse. Genau wie bei den Aminocarbonsäuren können die verwendeten Aminosulfonsäuren, welche ebenfalls praktisch vollständig in der Alkalimetallsalz-Form vorliegen, mehrere Aminogruppen und/oder Sulfonsäuregruppen aufweisen.

Als Carbonsäurehalogenide kommen vor allem Fettsäurechloride und -bromide, d.h. Chloride und Bromide von gesättigten oder ungesättigten C₆- bis C₃₀-Monocarbonsäuren, in Betracht. Insbesondere eignen sich die gesättigten oder einfach bis mehrfach ungesättigten C₈- bis C₂₀-Monocarbonsäuren, z.B. Laurinsäure, Myristinsäure, Palmitinsäure, Stearinsäure, Ölsäure, Linolsäure oder Linolensäure, oder Gemische solcher natürlich vorkommenden langkettigen Fettsäuren, z. B. Kokosfettsäure, Palmölfettsäure, Palmkernölfettsäure, Talgfettsäure, Sojaölfettsäure, Leinölfettsäure, Canolafettsäure, Sonnenblumenölfettsäure oder Rapsölfettsäure, sowie entsprechende synthetisch erzeugte C₈- bis C₂₀-Monocarbonsäuren oder deren Gemische.

Als Phasentrennmittel können Ketone (wie z.B. Methylethylketon, Isopropylmethylketon, Aceton), Ester (wie z.B. Essigsäureethylester, Essigsäuremethylester, Propionsäureethylester, Propionsäuremethylester, Isopropylacetat und andere Carbonsäurealkyl- oder -arylester), Alkohole (wie z.B. Propanol, Isopropanol, n-Butanol, 2-Butanol, Isobutanol), Dialkylether (wie z.B. Diethylether, Methyl-tert.butylether, Diisopropylether) sowie andere, gemäß dem Stand der Technik hierfür bekannte und geeignete Verbindungen eingesetzt werden.

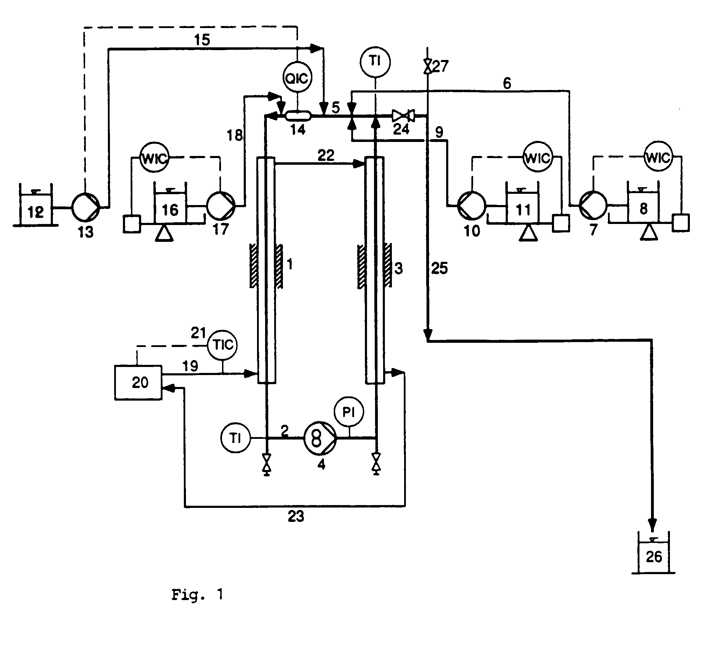

Die anliegende Zeichnung zeigt
- in Fig. 1: ein Verfahrensschema zur Erläuterung der Durchführung des erfindungsgemäßen Verfahrens bzw. der eingesetzten Vorrichtung.

Weitere Einzelheiten und Vorteile der Erfindung können dem anhand des in der Zeichnung dargestellten Verfahrensschemas beschriebenen Ausführungsbeispiel entnommen werden. Dabei soll das Funktionsprinzip zunächst allgemein am Beispiel der Herstellung von Oleoylsarkosin erläutert werden.

Der Reaktor für die Durchführung des erfindungsgemäßen Verfahrens besteht aus einem Pumpenumwälzkreis. Die Umwälzleitung des Reaktors ist aus verschiedenen Rohrstücken 1, 2, 3, 5 zusammengesetzt. Über Zuleitung 6 werden mit Pumpe 7 Ölsäurechlorid aus einem Behälter 8 und über Leitung 9 mit Pumpe 10 aus Behälter 11 Sarkosin-Natrium-Lösung jeweils mengengeregelt dem Reaktor zudosiert. Mittels Umwälzpumpe 4 wird die Umwälzströmung im Reaktor aufrechterhalten. Diese Rohrströmung gewährleistet eine effektive Durchmischung der Reaktanden. Für Anlagen mit größeren Rohrquerschnitten können die Rohre mit Einbauten wie z.B. statischen Mischern, Schüttungen, Packungen oder anderen zur Intensivierung des Mischens geeigneten Apparaten/Einbauten versehen werden. Alkalische Lösung, z.B. wäßrige NaOH-Lösung wird aus einem Behälter 12 mittels einer Pumpe 13 mit Hilfe einer pH-Meßsonde 14 über die Leitung 15 dosiert zugeführt, so daß ein konstanter pH-Wert im Reaktionsgemisch eingehalten wird. Des weiteren wird - bei Bedarf - ein anderes Reaktionsmittel wie ein organisches Phasentrennmittel z.B. Methylethylketon aus einem Behälter 16 über eine Pumpe 17 durch eine Leitung 18 mengengeregelt eingespeist. Die Verweilzeit im Umpumpkreislauf kann durch die Dosierrate gesteuert werden, die Durchmischung durch Variation des Umwälzstromes. Da es sich bei dem Kreislauf um ein vollständig mit Flüssigkeit gefülltes System handelt, tritt kein Schäumen der wäßrigen Tensidmischung auf.

Die Temperatur im Umpumpkreislauf wird geregelt. Bevorzugt erfolgt dies so, daß mindestens ein Teilstück des Reaktors, insbesondere die vor und nach der Umwälzpumpe 4 angeordneten Teilstücke des Reaktors, kühlbar ist. Die Wärmeabfuhr erfolgt über die jeweils mit Doppelmantel ausgestatteten Rohrstücke 1 und 3. Diese Doppelmäntel werden mit Kühlflüssigkeit über Leitung 19, 22 und 23 durchströmt. Die Temperatur des Kühlmediums kann in Abhängigkeit von der gewünschten Produkttemperatur eingestellt werden. Der entsprechende Regelkreis erhält über die als TIC bezeichnete Temperaturmeßstelle 21 den jeweiligen Ist-Wert der Kühlmittelvorlauftemperatur. Die Einstellung erfolgt mittels Thermostat 20. Eine Regelung über eine direkte Produkttemperaturmessung ist möglich. Das Abführen der Reaktionswärme in größeren Anlagen kann gleichfalls durch den Einbau eines üblichen Wärmetauschers wie z.B. Plattenwärmetauscher, Rohrbündelwärmetauscher oder ähnlichen Apparaten in die als Umwälzkreis ausgeführte Rohrleitung erfolgen. Die Kühlflüssigkeit fließt nach Verlassen des Kühlmantels des Teilstücks 1 über eine Leitung 22 in den Kühlmantel des Teilstücks 3 und von diesem über die Leitung 23 wieder zurück zum Thermostaten 20. Der kontinuierliche Austrag eines Teilproduktstromes aus dem Umpumpkreislauf erfolgt durch ein Überströmventil 24 über die Austragsleitung 25, die in einen Behälter 26 mündet. An die Austragsleitung 25 ist eine Entlüftungsleitung 27 angeschlossen. Mit TI sind in der Zeichnung weitere Temperaturmeßstellen, mit PI ist eine Druckmeßstelle bezeichnet, WIC und QIC bedeuten Gewichtsmeßstelle und Durchflußmeßstelle.

Das Volumen des Umpumpkreislaufs in der für die Durchführung der Beispiele 1 - 3 verwendeten Vorrichtung durch den Reaktor betrug 170 ml. Die Abmessungen der Reaktorrohre 1 und 3 waren ⌀ 12 x 1 x 1000 mm.

Die Dosierung und der Produktaustrag erfolgen kontinuierlich und können über ein Prozeßleitsystem gesteuert werden. Ist das System im Gleichgewicht, so findet die Acylierungsreaktion stets in einem in seiner Zusammensetzung konstanten Reaktionsmedium statt. D.h. reaktionsrelevante Parameter wie Viskosität, Konzentration etc. ändern sich nicht. Damit bleibt auch der Wärmeübergang konstant, wodurch eine gleichmäßige, sehr gut regelbare Wärmeabfuhr gewährleistet wird.

An diese kontinuierliche Acylierung schließen sich Neutralisation, Phasentrennung und Entfernung des organischen Lösungsmittels an, wobei der den Reaktor verlassende Produktstrom über weitere Reaktoren zur Nach- und Weiterreaktion geführt werden kann. Diese können in bekannter Weise ebenfalls kontinuierlich durchgeführt werden. Hierbei kann die Neutralisation über eine Mischstrecke, die Phasentrennung über ein einfaches Settler-System und das Entfernen des Lösungsmittels über Verdampfer erfolgen.

Mit der vorstehend beschriebenen Versuchsanordnung wurden folgende Versuchsbeispiele für die Synthese von N-Oleoylsarkosin durchgeführt.

### BEISPIEL 1

Das Volumen des Umpumpkreises (170 ml) wird mit Wasser oder einer wäßrigen Lösung aus Oleoylsarkosin-Na und Methylethylketon gefüllt.

Bei einer Temperatur von 40°C werden gleichzeitig 206 g/h Wasser, 211 g/h einer Sarkosin-Na-Lösung (40,1 %ig), 229 g/h Ölsäurechlorid sowie 94 g/h Methylethylketon zugefahren. Über die in den Kreislauf eingebaute pH-Elektrode wird der pH-Wert gemessen, und durch Zugabe von 10%iger Natronlauge wird der pH-Wert konstant zwischen 10 und 10,5 gehalten.

Über das Überströmventil wird ein Teil des fertigen Rohproduktes als wäßrige Suspension in Wasser und Methylethylketon zusammen mit dem entstandenen NaCl (in der Suspension gelöst) kontinuierlich aus dem Umpumpkreis abgelassen. Die Umwälzpumpe, die das Reaktionsgemisch im Kreis pumpt, wird so eingestellt, daß ein Fluß von 1200 ml/min erreicht wird. Die mittlere Verweilzeit beträgt 10 Minuten.

Zur Aufarbeitung wird das aus dem Umpumpkreis ausgeschleuste Rohgemisch anschließend kontinuierlich durch Zugabe von konz. Schwefelsäure auf pH 1 - 1,5 eingestellt. In einem nachfolgend geschalteten Phasenscheider erfolgt die Phasentrennung. Die organische Phase wird über einen Fallfilmverdampfer geleitet und vom Phasentrennmittel (Methylethylketon) befreit.

Man erhält die freie Oleoylsarkosinsäure als hellgelbes Öl (255 g/h) mit einem Gehalt von 96,4 Gew.-% Oleoylsarkosinsäure und 3,6 Gew.-% Ölsäure.

### BEISPIEL 2

Das Volumen des Umpumpkreises (170 ml) wird mit Wasser oder einer wäßrigen Lösung aus Oleoylsarkosin-Na und Methylethylketon gefüllt.

Bei einer Temperatur von 40°C werden gleichzeitig 425 g/h Wasser, 235 g/h einer Sarkosin-Na-Lösung (40,1 %ig), 213 g/h Ölsäurechlorid sowie 91 g/h Methylethylketon zugefahren. Über die in den Kreislauf eingebaute pH-Elektrode wird der pH-Wert gemessen, und durch Zugabe von 50%iger Natronlauge wird der pH-Wert konstant zwischen 10 und 10,5 gehalten.

Über das Überströmventil wird ein Teil des fertigen Rohproduktes als wäßrige Suspension in Wasser und Methylethylketon zusammen mit dem entstandenen NaCl (in der Suspension gelöst) kontinuierlich aus dem Umpumpkreis abgelassen. Die Umwälzpumpe, die das Reaktionsgemisch im Kreis pumpt, wird so eingestellt, daß ein Fluß von 1200 ml/min erreicht wird. Die mittlere Verweilzeit beträgt 10 Minuten.

Zur Aufarbeitung wird das Rohgemisch anschließend in einem Pufferbehälter durch Zugabe von konz. Schwefelsäure auf pH 1 - 1,5 eingestellt. In einem nachfolgend geschalteten Phasenscheider erfolgt die kontinuierliche Phasentrennung. Die organische Phase wird über einen Fallfilmverdampfer geleitet und vom Phasentrennmittel (Methylethylketon) befreit.

Man erhält die freie Oleoylsarkosinsäure als hellgelbes Öl (248 g/h) mit einem Gehalt von 96,5 Gew.-% Oleoylsarkosinsäure und 3,5 Gew.-% Ölsäure.

### BEISPIEL 3

Das Volumen des Umpumpkreises (170 ml) wird mit Wasser oder einer wäßrigen Lösung aus Oleoylsarkosin-Na und Methylethylketon gefüllt.

Bei einer Temperatur von 30°C werden gleichzeitig 438 g/h Wasser, 211 g/h einer Sarkosin-Na-Lösung (40,1 %ig), 220 g/h Ölsäurechlorid sowie 94 g/h Methylethylketon zugefahren. Über die in den Kreislauf eingebaute pH-Elektrode wird der pH-Wert gemessen, und durch Zugabe von 50%iger Natronlauge wird der pH-Wert konstant zwischen 10 und 10,5 gehalten.

Über das Überströmventil 24 wird ein Teil des fertigen Rohproduktes als wäßrige Suspension in Wasser und Methylethylketon zusammen mit dem entstandenen NaCl (in der Suspension gelöst) kontinuierlich aus dem Umpumpkreis abgelassen. Die Umwälzpumpe, die das Reaktionsgemisch im Kreis pumpt, wird so eingestellt, daß ein Fluß von 1200 ml/min erreicht wird. Die mittlere Verweilzeit beträgt 10 Minuten.

Zur Aufarbeitung wird das Rohgemisch anschließend kontinuierlich durch Zugabe von konz. Schwefelsäure auf pH 1 - 1,5 eingestellt. In einem nachfolgend geschalteten Phasenscheider erfolgt die kontinuierliche Phasentrennung. Die organische Phase wird über einen Fallfilmverdampfer geleitet und vom Phasentrennmittel (Methylethylketon) befreit.

Man erhält die freie Oleoylsarkosinsäure als hellgelbes Öl (252 g/h) mit einem Gehalt von 92 Gew.-% Oleoylsarkosinsäure und 5,2 Gew.-% Ölsäure.

### VERGLEICHSBEISPIEL V 1

### Diskontinuierliche Herstellung von Oleoylsarkosin in einer 1000 ml Rührapparatur

In einer 1000 ml Rührapparatur werden 275 ml Wasser vorgelegt und mit 138,5 g Sarkosin-Na-Lösung (40,1 %ig in Wasser) und 65 g Methylethylketon versetzt. Anschließend werden gleichzeitig bei 30°C 151,8 g Ölsäurechlorid und 40,5 g einer 50%igen Natronlauge so zugetropft, daß die Temperatur bei 30°C und der pH-Wert der Reaktionsmischung bei 10 - 10,5 gehalten wird. Nach beendeter Zugabe (ca. 30 min) wird der pH-Wert des Reaktionsgemisches mit 35 g konzentrierter Schwefelsäure auf 1,5 gestellt. Die organische Phase wird im Scheidetrichter von der wäßrigen Phase abgetrennt und am Rotationsverdampfer bei ca. 50°C und reduziertem Druck vom Lösungsmittel befreit (Dauer ca. 60 min).

Man erhält 164,9 g eines orangegelben Öls, Gehalt an N-Oleoylsarkosin: 92,5%.

Vergleicht man die erfindungsgemäßen Beispiele 1 - 3 mit dem nach dem Stand der Technik durchgeführten Vergleichsbeispiel V1, so zeigt sich, daß mit dem erfindungsgemäßen Verfahren erheblich höhere Ausbeuten pro Zeit- und Volumeneinheit erzielt werden können, als dies bei der bekannten Verfahrensweise der Fall ist.

Im Vergleichsbeispiel wurde in der Acylierungsstufe in einem Reaktorvolumen von 1000 ml gearbeitet. Es wurden in 90 Minuten 164,9 g Produkt hergestellt.

In den Beispielen 1 - 3 wurde in einem Reaktorvolumen von 170 ml gearbeitet. Es wurden pro Stunde ca. 255 g Produkt hergestellt.

Dies bedeutet, daß mit dem erfindungsgemäßen Verfahren pro Zeiteinheit mehr als die doppelte Menge, pro Volumeneinheit mehr als die neunfache Menge an Oleoylsarkosin hergestellt werden kann, und somit ein Verfahren vorliegt, mit dem auch großtechnisch in guter Raum/Zeit-Ausbeute Acylsarkosine und andere Acylaminosäuren hergestellt werden können.

Des weiteren konnte mit den Beispielen 1 - 3 gezeigt werden, daß mit dem erfindungsgemäßen Verfahren Oleoylsarkosin in sehr guter Ausbeute und hoher Reinheit erhalten werden kann.

## Patentansprüche

1. Verfahren zur kontinuierlichen Herstellung von N-Acylaminocarbonsäuren und N-Acylaminosulfonsäuren sowie deren Alkalimetallsalzen aus den Alkalimetallsalzen von Aminocarbonsäuren bzw. Aminosulfonsäuren und Carbonsäurehalogeniden in einem als Umwälzkreis ausgebildeten Reaktor, dadurch gekennzeichnet, daß die Reaktanden zur sofortigen Reaktion in den Umwälzkreis eingespeist und ein der Zulaufmenge entsprechender Teil der Produktlösung kontinuierlich aus dem Umwälzkreis ausgeschleust wird, die Reaktanden durch eine Umwälzpumpe im Kreislauf geführt werden, die bei der Reaktion gebildete Wärme abgeführt und die Temperatur in dem Reaktor geregelt wird.

2. Verfahren nach Anspruch 1, dadurch gekennzeichnet, daß dem Umwälzkreis zur Einhaltung eines konstanten pH-Werts pH-gesteuert alkalische Lösung zudosiert wird.

3. Verfahren nach Anspruch 1 oder 2, dadurch gekennzeichnet, daß in den Umwälzkreis ein organisches Phasentrennmittel eingespeist wird.

4. Verfahren nach einem der Ansprüche 1 bis 3, dadurch gekennzeichnet, daß der den Reaktor verlassende Produktstrom über weitere Reaktoren zur Nach- oder Weiterreaktion geführt wird.

## Claims

1. A process for the continuous preparation of N-acylamino carboxylic acids and N-acylamino sulfonic acids, and their alkali metal salts, from the alkali metal salts of amino carboxylic acids and amino sulfonic acids, respectively, and carbonyl halides in a reactor designed as circulating circuit, wherein the reactants are fed for immediate reaction into the circulating circuit, and a part of the product solution corresponding to the amount fed in is continuously discharged from the circulating circuit, the reactants are circulated by a circulating pump, the heat produced in the reaction is removed and the temperature in the reactor is controlled.

2. A process as claimed in claim 1, wherein an alkaline solution is metered under pH control into the circulating circuit to maintain a constant pH.

3. A process as claimed in claim 1 or 2, wherein an organic agent to induce phase separation is fed into the circulating circuit.

4. A process as claimed in any of claims 1 to 3, wherein the product stream leaving the reactor is passed through further reactors for subsequent or further reaction.

## Revendications

1. Procédé de préparation continue d'acides N-acylaminocarboxyliques et d'acides N-acylaminosulfoniques, ainsi que de leurs sels de métaux alcalins, à partir des sels de métaux alcalins d'acides aminocarboxyliques ou aminosulfoniques et d'halogénures d'acides carboxyliques, dans un réacteur configuré en circuit de circulation, caractérisé en ce que les réactifs sont introduits dans le circuit de circulation aux fins de réaction immédiate, et qu'une partie de la solution de produit correspondant à la quantité d'alimentation est soutirée en continu du circuit de circulation, que les réactifs sont introduits dans le circuit par une pompe de circulation, que la chaleur générée par la réaction est évacuée et que la température est régulée au sein du réacteur.

2. Procédé selon la revendication 1, caractérisée en ce qu'une solution alcaline à régulation de pH est introduite dans le circuit de circulation pour y maintenir un pH constant.

3. Procédé selon la revendication 1 ou 2, caractérisé en ce qu'un agent organique de séparation de phases est introduit dans le circuit de circulation.

4. Procédé selon l'une des revendications 1 à 3, caractérisé en ce que le courant de produit quittant le réacteur est envoyé à d'autres réacteurs pour post-réaction ou réaction complémentaire.
